(19) Europäisches Patentamt
European Patent Office
Office européen des brevets

(11) Veröffentlichungsnummer: **0 152 022**
**B1**

(12) # EUROPÄISCHE PATENTSCHRIFT

(45) Veröffentlichungstag der Patentschrift: **05.10.88**

(51) Int. Cl.⁴: **C 07 C 149/36**

(21) Anmeldenummer: **85101005.8**

(22) Anmeldetag: **31.01.85**

(54) **Di-(substituiertes hydroxyphenylthio)-alkane und -cycloalkane und sie enthaltende Stoffzusammensetzungen.**

(30) Priorität: **03.02.84 US 576691**

(43) Veröffentlichungstag der Anmeldung: **21.08.85 Patentblatt 85/34**

(45) Bekanntmachung des Hinweises auf die Patenterteilung: **05.10.88 Patentblatt 88/40**

(84) Benannte Vertragsstaaten: **DE FR GB IT**

(56) Entgegenhaltungen:
**FR - A - 1 561 853**
**GB - A - 2 075 519**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Spivack, John D., 1 Blue Jay Street, Spring Valley New York 10977 (US)**
Erfinder: **Pastor, Stephen D., 1080 Warburton Avenue, Yonkers New York 10701 (US)**

(74) Vertreter: **Zumstein, Fritz, Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft Alkane oder Cycloalkane, die zweimal durch eine substituierte Hydroxyphenylthiogruppe substituiert sind und ihre Verwendung als Stabilisatoren für organisches Material.

Organische polymere Verbindung wie Kunststoffe oder Harze unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophoren führen. Weiterhin müssen Probleme der Verarbeitungsstabilität und der Substratverträglichkeit beachtet werden.

Überraschenderweise ist jetzt gefunden worden, dass die zweimal durch eine Hydroxyphenylthiogruppe substituierten Alkane oder Cycloalkane dieser Erfindung eine ungewöhnliche Kombination wünschenswerter Eigenschaften besitzen, wodurch diese Stoffe zu besonders effektiven Stabilisatoren werden. Die erfindungsgemässen Verbindungen erweisen sich als besonders nützliche Stabilisatoren für Polyolefine, schlagfestes Polystyrol, Elastomere wie Polybutadien oder Styrol-Butadien-Elastomere, bei denen der Erhalt der Elastizität und die Verhinderung von Vernetzungsreaktionen, von Versprödung, von Verfärbung, von Geruchsbildung und von Ausschwitzen des Stabilisators grundlegende Qualitätsanforderungen darstellen.

Eine Reihe verwandter US-Patente der Nummern 3 576 883, 3 786 100, 3 897 500 und 3 956 359 beschreiben ketosubstituierte Alkylidendithiobisphenole, die durch das Vorhandensein von Bis-(3,5-di-tert.-butyl-4-hydroxyphenyl)-Gruppen und verschiedener Alkylidenbrücken charakterisiert sind. In jedem Fall muss ein Methylrest am Alkylidenbrückenglied der genannten Verbindungen gebunden sein. Die Verbindungen der obengenannten Patente sind als zur Senkung des Cholesterins im Blut von Warmblütlern geeignete Substanzen beschrieben. Kein Hinweis ist zu finden für deren Verwendung als Stabilisatoren. M.B. Neuworth et al., J. Medicinal Chemistry, 13, 722 (1970) beschreibt dieselben in den 4 oben erwähnten US-Patenten offenbarten Verbindungen sowie einige ähnliche Alkylidendithiophenole mit verschiedenen Substituenten am Phenylring und am Alkylidenbrückenglied. Auch in diesem Fall wird einzig und allein auf die cholesterinsenkende Wirkung dieser Substanzen hingewiesen. Drei Japanische Offenlegungsschriften, JP Patent Kokai 79/163536, 80/9041 und 80/17316 (entsprechend CA, 93, 71280x (1980); CA 93, 94980q (1980) und CA, 93, 149970u (1980)) beschreiben 2,2-Bis-(3,5-di-tert.-butyl-4-hydroxyphenylthio)-propan ebenfalls als cholesterinsenkendes Mittel.

US-Patent 3 704 327 beschreibt Bis-(3-methyl-5-tert.-butyl-4-hydroxyphenylthio)methan und 1,1-Bis-(3-methyl-5-tert.-butyl-4-hydroxyphenylthio)-ethan als Antioxidans für Kautschuk. Die erfindungsgemässen Verbindungen unterscheiden sich strukturell von diesen vorbekannten Verbindungen dadurch, dass sie in jedem Phenolring durch zwei tert.-Butyl-Gruppen in ortho-Stellung zur Hydroxygruppe substituiert sind und dass die Substituenten am Alkylidenbrückenglied zusammen mindestens fünf Kohlenstoffatome besitzen. Sie gewährleisten überraschend bessere Stabilisierungseffekte in verschiedenen Substraten im Vergleich zu den zwei obenerwähnten Verbindungen von US Patent 3 704 327.

FR-A-1 561 853 beschreibt Bis[3,5-di-tert-butyl-4-hydroxyphenylthio]-alkylidenderivate, wobei die Alkylidengruppe 3 bis 5 Kohlenstoffatome besitzt, und u.a. deren Verwendung als Stabilisatoren für Kunststoffe.

GB-A-2 075 519 offenbart Bis[3,5-di-tert-butyl-4-hydroxyphenylthio]alkylenderivate sowie deren Verwendung als Stabilisatoren für hochmolekulare Polyester.

Die vorliegende Erfindung betrifft demnach eine Stoffzusammensetzung enthaltend ein gegen oxidativen, thermischen oder strahlungsinduzierten Abbau empfindliches organisches Material und eine stabilisierende Menge einer Verbindung der Formel I

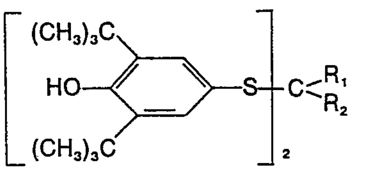

(I)

worin $R_1$ Wasserstoff oder $C_1$–$C_4$Alkyl ist, $R_2$ $C_6$–$C_8$Alkyl, Cyclohexyl, Cyclohex-3-enyl, Phenyl oder 3,5-Di-tert.-butyl-4-hydroxyphenyl bedeutet oder $R_1$ und $R_2$ zusammen $C_5$–$C_{11}$Alkylen bilden.

$R_1$ ist als $C_1$–$C_4$Alkyl geradkettiges oder verzweigtes Alkyl wie z.B. Methyl, Ethyl, Isopropyl, n-Propyl, Isobutyl, tert.-Butyl und dergleichen.

$R_2$ ist als $C_6$–$C_8$Alkyl geradkettiges oder verzweigtes Alkyl wie z.B. n-Hexyl, 2,4-Dimethylamyl, 3-Heptyl und dergleichen.

Wenn $R_1$ und $R_2$ zusammen $C_5$–$C_{11}$Alkylen bilden, so handelt es sich z.B. um Pentamethylen, Hexamethylen, Heptamethylen oder Undecamethylen und dergleichen.

$R_1$ ist bevorzugt Wasserstoff oder Methyl. Wenn $R_1$ und $R_2$ zusammen Alkylen bilden, so handelt es sich bevorzugt um Pentamethylen.

Viele der erfindungsgemäss einzusetzenden Verbindungen sind aus den obenerwähnten Publikationen bereits bekannt.

Verbindungen der Formel I, worin $R_1$ Wasserstoff und $R_2$ Cyclohexyl, Cyclohex-3-enyl oder 3,5-Di-tert.-butyl-4-hydroxyphenyl bedeuten sind aber neu und stellen einen weiteren Gegenstand der vorliegenden Erfindung dar.

Die erfindungsgemäss einzusetzenden Verbindungen können in Analogie zu allgemein bekannten Methoden durch Umsetzung eines geeigneten 4-Mercaptophenols mit der entsprechenden Carbonylverbindung in Gegenwart einer starken Säure, wie z.B. Chlorwasserstoff, als Katalysator hergestellt werden. Die Reaktion wird zweckmässig in einem inerten organischen Lösungsmittel, wie beispielsweise Methanol, Heptan, Benzol oder Toluol bei Temperaturen zwischen 0 und 100 °C durchgeführt. Die Ausgangsprodukte sind bekannt und im allgemeinen im Handel erhältlich. Sollten einige noch neu sein, so können sie in Analogie zu allgemein bekannten Verfahren hergestellt werden.

Die Verbindungen der Formel I lassen sich als effektive Stabilisatoren für Kunststoffe, Polymere oder Harze einsetzen. Sie lassen sich auch als Stabilisatoren in Mineralölen oder synthetischen Ölen, z.B. Schmieröle und Umlauföle, verwenden.

Vorzugsweise verwendet man die Verbindungen der Formel I als Stabilisatoren für homopolymere oder copolymere Polyolefine sowie für Polystyrol, einschliesslich schlagfestem Polystyrol, ABS-Harz, SBR, Polyisopren, natürlicher Kautschuk, Polyester, einschliesslich Polyethylenterephthalat und Polybutylenterephthalat und Copolymere sowie für Schmiermittel, wie beispielsweise die aus Mineralölen gewonnenen.

Polymere, die mit den erfindungsgemässen Verbindungen stabilisiert werden können, sind beispielsweise

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrlnitril-Methylacrylat; Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw., Alkylmethacrylate und Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z.B. Ethylenoxid enthalten.

13. Polyphenyloxide und -sulfide und deren Mischungen mit Styrolpolymeren.

14. Polyurethane, die sich von Polyethern, Polyestern und Polybutadienen mit enständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z.B. mit Polyethylenglykol, Polypropylenglykol oder Polytetramethylenglykol.

16. Polyharnstoffe, Polyimide, Polyamid-imide und Polybenzimidazole.

17. Polyester, sie sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-di-methylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z.B. von Bis-glycidylethern oder von cycloaliphatischen Diepoxiden.

29. Natürliche Polymere, wie Cllulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z.B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Spinnpräparationen Anwendung finden, sowie deren wässrigen Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die erfindungsgemässen Stabilisatoren werden dem zu stabilisierenden Material in Mengen von 0,01 bis 5,0 Gew.-% bezogen auf das stabilisierte Material zugemischt. Je nach Verwendung und Substrat wird die Stabilisatormenge variiert. Bevorzugt verwendet man 0,05 bis 2 Gew.-% des Stabilisators, besonders bevorzugt 0,1 bis 1 Gew.-%.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit den pulverförmigen Polymeren gemischt werden, oder eine Emulsion bzw. eine Suspension des Stabilisators kann mit einer Lösung, Suspension oder Emulsion des Polymeren vermischt werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

In der Praxis können die Verbindungen der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z.B. weitere aminische Antioxidantien, Metallpassivatoren, Rostinhibitoren, Viskositäts-Index Verbesserer, Stockpunkterniedriger, Dispergiermittel/Tenside und Verschleissschutzadditive.

Als weitere Additive, mit denen zusammen die erfindungsgemäss verwendeten Stabilisatoren eingesetzt werden können, sind beispielsweise zu nennen:

1. Antioxidantien
1.1. Alkylierte Monophenole
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone
2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether
2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole
2,2′-Methylen-bis-(6-tert.butyl-4-methylphenol)
2,2′-Methylen-bis-(6-tert.butyl-4-ethylphenol)
2,2′-Methylen-bis-[4-methyl-6-(α-methylcyclohexyl)-phenol]
2,2′-Methylen-bis-(4-methyl-6-cyclohexylphenol)
2,2′-Methylen-bis-(6-nonyl-4-methylphenol)
2,2′-Methylen-bis-(4,6-di-tert.butylphenol)
2,2′-Ethyliden-bis-(4,6-di-tert.butylphenol)
2,2′-Ethyliden-bis-(6-tert.butyl-4-isobutylphenol)
2,2′-Methylen-bis-[6-(α-methylbenzyl)-4-nonylphenol]
2,2′-Methylen-bis-[6-(α,α-dimethylbenzyl)-4-nonylphenol]
4,4′-Methylen-bis-(2,6-di-tert.butylphenol)
4,4′-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercapto-butan
Ethylenglycol-bis-[3,3-bis-(3′-tert.butyl-4′-hydroxyphenyl)-butyrat]
Di-(3-tert.butyl-4-hydroxy-5-methylphenyl)-dicyclopentadien
Di-[2-(3′-tert.butyl-2′-hydroxy-5′-methylbenzyl)-6-tert.butyl-4-methyl-phenyl]-terephthalat.

1.5 Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)-dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester,
Calcium-salz.

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit
Methanol
Octadecanol
1,6-Hexandiol
Neopentylglycol
Thiodiethylenglycol
Diethylenglycol
Triethylenglycol
Pentaerythrit
Tris-hydroxyethyl-isocyanurat
Di-hydroxyethyl-oxalsäurediamid

1.8. Ester der β-(5-tert.butyl-4-hydroxy-3-methylphenyl)-propionsäure mit ein- oder mehrwertigen Alkoholen, wie z.B. mit
Methanol
Octadecanol
1,6-Hexandiol
Neopentylglycol
Thiodiethylenglycol
Diethylenglycol
Triethylenglycol
Pentaerythrit
Tris-hydroxyethyl-isocyanurat
Di-hydroxyethyl-oxalsäurediamid

1.9. Amide der β-(3,5-Di-tert.butyl-4-hydroxyphenyl)-propionsäure, wie z.B.
N,N′-Di-(3,5-di-tert.butyl-4-hydroxyphenylpropionyl)-hexamethylendiamin
N,N′-Di-(3,5-di-tert.butyl-4-hydroxyphenylpropionyl)-trimethylendiamin
N,N′-Di-(3,5-di-tert.butyl-4-hydroxyphenylpropionyl)-hydrazin

2. UV-Absorber und Lichtschutzmittel
2.1. 2-(2′-Hydroxyphenyl)-benztriazole, wie z.B. das
5′-Methyl-, 3′,5′-Di-tert.butyl-,
5′-Tert.butyl-,
5′-(1,1,3,3-Tetramethyl-butyl)-,
5-Chlor-3′,5′-di-tert.butyl-,
5-Chlor-3′-tert.butyl-5′-methyl-,
3′-sec.Butyl-5′-tert.butyl,
4′-Octoxy-, 3′,5′-Di-tert.amyl-,
3′,5′-Bis-(α,α-dimethylbenzyl)-Derivat.

2.2. 2-Hydroxybenzophenone, wie z.B. das
4-Hydroxy-, 4-Methoxy-, 4-Octoxy-,
4-Decyloxy-, 4-Dodecyloxy-, 4-Benzyloxy-,
4,2′,4′-Tri-hydroxy-,
2′-Hydroxy-4,4′-dimethoxy-Derivat.

2.3. Ester von gegebenenfalls substituierten Benzoesäuren, wie z.B.
4-Tert.butyl-phenylsalicylat,
Phenylsalicylat, Octylphenylsalicylat,
Dibenzoylresorcin,
Bis-(4-tert.butylbenzoyl)-resorcin,
Benzoylresorcin,
3,5-Di-tert.butyl-4-hydroxybenzoesäure-2,4-di-tert.butylphenylester,
3,5-Di-tert.butyl-4-hydroxybenzoesäure-hexadecylester.

2.4. Acrylate, wie z.B.
α-Cyan-β,β-diphenylacrylsäure-ethylester
bzw. -isooctylester,
α-Carbomethoxy-zimtsäuremethylester,
α-Cyano-β-methyl-p-methoxy-zimtsäuremethylester
bzw. -butylester,
α-Carbomethoxy-p-methoxy-zimtsäure-methylester,
N-(β-Carbomethoxy-β-cyanovinyl)-2-methyl-indolin

2.5. Nickelverbindungen, wie z.B. Nickelkomplexe des
2,2′-Thio-bis-[4-(1,1,3,3-tetramethylbutyl)-phenols], wie der 1 : 1- oder der 1 : 2-Komplex, gegebenenfalls mit zusätzlichen Liganden wie n-Butylamin, Triethanolamin oder N-Cyclohexyldiethanolamin, Nickeldibutyldithiocarbamat, Nikkelsalze von
4-Hydroxy-3,5-di-tert.butylbenzyl-phosphonsäure-monoalkylestern wie vom Methyl- oder Ethylester, Nickelkomplexe von Ketoximen wie von
2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B.
Bis-(2,2,6,6-tetramethyl-piperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester,
Kondensationsprodukt aus
1-Hydroxyethyl-2,2,6,6-tetramethyl-4-hydroxypiperidin und Bernsteinsäure, Kondensationsprodukt aus
N,N′-(2,2,6,6-Tetramethyl-4-piperidyl)-hexamethylendiamin und 4-tert.Octylamino-2,6-dichlor-1,3,5-s-triazin,
Tris-(2,2,6,6-tetramethyl-4-piperidyl)-nitrilotriacetat,
Tetrakis-(2,2,6,6-tetramethyl-4-piperidyl)-1,2,3,4-butantetracarbonsäure,
1,1′-(1,2-Ethandiyl)-bis-(3,3,5,5-tetramethylpiperazinon).

2.7. Oxalsäurediamide, wie z.B.
4,4′-Di-octyloxy-oxanilid,
2,2′-Di-octyloxy-5,5′-di-tert.butyl-oxanilid,
2,2′-Di-dodecyloxy-5,5′-di-tert.butyl-oxanilid,
2-Ethoxy-2′-ethyl-oxanilid,
N,N′-Bis-(3-dimethylaminopropyl)-oxalamid,
2-Ethoxy-5-tert.butyl-2′-ethyloxanilid
und dessen Gemisch mit
2-Ethoxy-2′-ethyl-5,4′-di-tert.butyl.oxanilid,
Gemische von ortho- und para-Methoxy- sowie von o- und p-Ethoxy-di-substituierten Oxaniliden.

3. Metalldesaktivatoren, wie z.B.
N,N′-Diphenyloxalsäurediamid,
N-Salicylal-N′-salicyloylhydrazin,
N,N′-Bis-salicyloylhydrazin,
N,N′-Bis-(3,5-di-tert.butyl-4-hydroxyphenylpropionyl)-hydrazin,
3-Salicyloylamino-1,2,4-triazol,
Bis-benzyliden-oxalsäuredihydrazid.

4. Phosphite und Phosphonite, wie z.B.
Triphenylphosphit,
Diphenylalkylphosphite,
Phenyldialkylphosphite,
Tri-(nonylphenyl)-phosphit,
Trilaurylphosphit, Trioctadecylphosphit,
Distearyl-pentaerythritdiphosphit,
Tris-(2,4-di-tert.butylphenyl)-phosphit,
Diisodecylpentaerythrit-diphosphit,
Di-(2,4-di-tert.butylphenyl)-pentaerythritdiphosphit,
Tristearyl-sorbit-triphosphit,
Tetrakis-(2,4-di-tert.butylphenyl)-4,4′-biphenylen-diphosphonit.

5. Peroxidzerstörende Verbindungen, wie z.B.
Ester der β-Thio-dipropionsäure,
beispielsweise der Lauryl-, Stearyl-,
Myristyl- oder Tridecylester,
Mercaptobenzimidazol,
das Zinksalz des 2-Mercaptobenzimidazols,
Zink-dibutyl-dithiocarbamat,
Dioctadecyldisulfit,
Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B.
Melamin, Polyvinylpyrrolidon,
Dicyandiamid, Triallylcyanurat,
Harnstoff-Derivate, Hydrazin-Derivate,
Amine, Polyamide, Polyurethane,
Alkali- und Erdalkalisalze
höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Für den Einsatz zusammen mit den erfindungsgemässen Verbindungen bevorzugte Costabilisatoren sind Antioxidantien, Lichtschutzmittel, Metalldesaktivatoren, Phosphite, Phosphonite und Thiosynergisten (peroxidzerstörende Verbindungen).

Die folgenden Beispiele erläutern die Erfindung. Alle Mengenangaben entsprechen Gewichtsteilen soweit nicht anderweitig angegeben.

Beispiel 1

2,2-Bis-(3,5-di-tert.-butyl-4-hydroxyphenylthio)octan

In einem Kolben wird unter Stickstoff eine Lösung von 23,84 g 2,6-Di-tert.butyl-4-mercaptophenol und 6,41 g 2-Octanon in 150 ml Methanol eingetragen und zwei Stunden bei 55 °C mit wasserfreiem Chlorwasserstoff behandelt. Der erhaltene Niederschlag wird abfiltriert und getrocknet. Man erhält 23,71 g (81% d.Th.) einer weissen festen Substanz mit Smp. 130–133 °C.

Analyse für $C_{36}H_{58}O_2S_2$

| | | |
|---|---|---|
| Berechnet: | C 73,7, | H 10,0 |
| Gefunden: | C 74,0, | H 9,8 |

Beispiel 2

1,1-Bis-(3,5-di-tert.-butyl-4-hydroxyphenylthio)-1-(3,5-di-tert.-butyl-4-hydroxyphenyl)-methan

Das unter Beispiel 1 beschriebene Verfahren wird wiederholt mit 23,84 g 2,6-Di-tert.butyl-4-mercaptophenol, 11,72 g 3,5-Di-tert.-butyl-4-hydroxybenzaldehyd und 150 ml Methanol. Durch Zermahlen mit Methanol erhält man 30 g (87% d.Th.) einer weissen kristallinen Substanz mit Smp. 175–177 °C.

Analyse für $C_{43}H_{64}O_3S_2$

| | | | |
|---|---|---|---|
| Berechnet: | C 74,5, | H 9,3, | S 9,3 |
| Gefunden: | C 74,5, | H 9,3, | S 9,5 |

Beispiel 3

4,4-Bis-(3,5-Di-tert.-butyl-4-hydroxyphenylthio)-2,6,8-trimethylnonan

Das unter Beispiel 1 beschriebene Verfahren wird wiederholt mit 9,22 g 2,6,8-Trimethylnonan-4-on, 23,84 g 2,6-Di-tert.-butyl-4-mercaptophenol und 150 ml Methanol. Das erhaltene Produkt wird chromatographisch gereinigt. Man erhält eine klare sirupöse Substanz, deren NMR-Spektrum die erwünschte Konstitution bestätigt.

Beispiel 4

α,α-Bis-(3,5-di-tert.-butyl-4-hydroxyphenylthio)-toluol

Das unter Beispiel 1 beschriebene Verfahren wird wiederholt mit 23,84 g 2,6-Di-tert.-butyl-4-mercaptophenol, 5,31 g Benzaldehyd und 150 ml Methanol. Durch Zermahlen des erhaltenen Produktes mit Methanol erhält man 18,74 g (60% d.Th.) einer weissen kristallinen Substanz mit Smp. 122–124 °C.

Analyse für $C_{35}H_{48}O_2S_2$

| | | |
|---|---|---|
| Berechnet: | C 74,4, | H 8,6 |
| Gefunden: | C 74,1, | H 8,6 |

Beispiel 5

1,1-Bis-(3,5-di-tert.-butyl-4-hydroxyphenylthio)-2-ethylhexan

Das unter Beispiel 1 beschriebene Verfahren wird wiederholt mit 23,84 g 2,6-Di-tert.-butyl-4-mercaptophenol, 6,41 g 2-Ethylhexanal und 150 ml Methanol. Das erhaltene Produkt wird chromatographisch gereinigt. Man erhält 10,29 g einer klaren sirupösen Substanz.

Analyse für $C_{35}H_{48}O_2S_2$

| | | |
|---|---|---|
| Berechnet: | C 73,7, | H 10,0 |
| Gefunden: | C 73,9, | H 9,9 |

Beispiel 6

1,1-Bis-(3,5-di-tert.-butyl-4-hydroxyphenylthio)-cyclohexan

Das unter Beispiel 1 beschriebene Verfahren wird wiederholt mit 28,71 g 2,6-Di-tert.-butyl-4-mercaptophenol, 5,91 g Cyclohexanon und 150 ml Methanol. Durch Zermahlen des erhaltenen Produkts mit Methanol erhält man 31,17 g (93,3% d.Th.) einer weissen, festen Substanz mit Smp. 195–197 °C.

Analyse für $C_{34}H_{52}O_2S_2$

| | | |
|---|---|---|
| Berechnet: | C 73,3, | H 9,4 |
| Gefunden: | C 73,4, | H 9,5 |

Beispiel 7

1,1-Bis-(3,5-di-tert.-butyl-4-hydroxyphenylthio)-1-(cyclohex-3-enyl)-methan

Eine Lösung von 10 g 2,6-Di-tert.-butyl-4-mercaptophenol und 2,3 g Cyclohex-3-enyl-carboxyaldehyd wird in einem mit Flamme getrockneten Kolben eingetragen und zwei Stunden unter Rühren mit 1,0 ml Tetrafluorborsäure behandelt. Das Reaktionsgemisch wird mit wässrigem Natriumbicarbonat extrahiert und über wasserfreiem Magnesiumsulfat getrocknet. Das Lösungsmittel wird im Vakuum abgedampft und der Rückstand aus Acetonitril umkristallisiert. Man erhält 9.0 g (76% d.Th.) einer weissen kristallinen Substanz mit Smp. 122–125 °C.

Analyse für $C_{35}H_{52}O_2S_2$

| | | | |
|---|---|---|---|
| Berechnet: | C 73,9, | H 9,2, | S 11,3 |
| Gefunden: | C 73,9, | H 9,3, | S 11,2 |

Beispiel 8

Stabilisierung von schlagfestem Polystyrol

Man stellt eine Lösung von 8 Gew.-% Polybutadien-Kautschuk (Firestone DIENE 55®) in monomerem Styrol her, indem man beide Komponenten in einer Quetschmühle mischt. Gleichzeitig wird in diesem Arbeitsgang der jeweils gewünschte Anteil des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, dass mit einem Wendelrührer ausgerüstet ist. Da die meisten IPS Blockpolymeriationen thermisch initiiert werden, wird in der vorliegenden Laboratoriumsversion kein Starter zugesetzt. Die Reaktion wird unter Stickstoff und unter Erwärmen durchgeführt. Der Reaktor wird innerhalb einer halben Stunde auf 121 °C erwärmt, und diese Temperatur wird aufrecht erhalten, bis zwischen 30 und 35% des Monomeren umgesetzt sind (Zeitdauer ca. 2,5 Stunden). Während der Polymerisation wird heftig gerührt, und die Rührgeschwindigkeit wird so eingestellt, dass Polymerpartikel von 2 bis 4 µm Grösse entstehen. Anschliessend werden die Reaktionsgefässe dem Reaktor entnommen, im Stickstoffstrom geöffnet und in ein Wirbelschichtsandbad gestellt, um die Polymerisation zu vervollständigen. Die Gefässe werden im Sandbad folgendermassen erhitzt:
eine Stunde bei 100 °C um die Anfangstemperatur vorzugeben, eine weitere Stunde, um 140 °C zu erreichen und anschliessend weitere 8 Stunden, wobei die Temperatur jede Stunde um 10 °C erhöht wird, bis schliesslich ein Maximum von 220 °C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200 °C für 45 Minuten bei einem Druck von 1,33 mbar belassen, um flüchtige Bestandteile zu entfernen. Nach dieser Behandlung wird der Block sofort in einer heizbaren hydraulischen Presse bei 205 °C zwischen zwei Aluminiumfolien zu einem dicken Stab gepresst (drei Minuten erhitzen, fünf Minuten ungeheizt).

Der Stab wird mittels einer Bandsäge zerkleinert und anschliessend granuliert. Alle Polymermischungen werden bei 205 °C extrudiert und dann zerkleinert. Die Pellets werden unter Druck bei 205 °C in dehnbare 3,2 mm dicke Streifen verformt. Diese Probestreifen werden dann bei 150 °C in einem Umluftofen gealtert, wobei sie auf Glasplatten, die sich wiederum auf rotierenden Trägern befinden, aufgebracht sind. Nach bestimmten Zeitintervallen wird der Yellowness Index der Proben gemäss ASTM D-1925-63T bestimmt, und es wird die Dehnbarkeit bestimmt (Instron Tensile Testing Apparatus, Instron Engineering Corporation, Massachusetts; Ziehgeschwindigkeit: 5 mm/Minute, ASTM D-638).

In den folgenden Tabellen 1 und 2 sind die Ergebnisse der Dehnbarkeitsmessungen und der Messungen des Yellowness-Index von bei 80 bzw. 150 °C ofengealterten Proben angegeben.

Tabelle 1:
Dehnbarkeitsmessungen und Yellowness Index von bei 80 °C ofengealterten Proben mit Stabilisator

| Additiv (Produkt vom Beispiel) | Stabilisator-menge (Gew.-%) | Dehnbarkeit der Probe (%) und Dauer der Hitzebelastung (h) | | | | |
|---|---|---|---|---|---|---|
| | | 0h | 300h | 600h | 900h | 1200h |
| keines | – | 33 | 9 | 3 | 3 | 3 |
| 1 | 0,1 | 55 | 24 | 10 | 9 | 7 |
| 7 | 0,1 | 49 | 29 | 10 | 8 | 5 |

| Additiv (Produkt vom Beispiel) | Stabilisator-menge (Gew.-%) | Yellowness Index und Dauer der Hitzebelastung (h) | | | | |
|---|---|---|---|---|---|---|
| | | 0h | 300h | 600h | 900h | 1200h |
| keines | – | 7 | 14 | 45 | 59 | – |
| 1 | 0,1 | 0 | 16 | 27 | 38 | 52 |
| 7 | 0,1 | 2 | 8 | 19 | 31 | 43 |

Tabelle 2:
Dehnbarkeitsmessungen und Yellowness Index von bei 150 °C ofengealterten Proben mit Stabilisator

| Additiv (Produkt vom Beispiel) | Stabilisator-menge (Gew.-%) | Dehnbarkeit der Probe (%) und Dauer der Hitzebelastung (h) | | | | |
|---|---|---|---|---|---|---|
| | | 0h | 1/2h | 1h | 1 1/2h | 2h |
| keines | – | 33 | 7 | 7 | 3 | 3 |
| 1 | 0,1 | 55 | 50 | 19 | 8 | 7 |
| 7 | 0,1 | 49 | 36 | 18 | 10 | 7 |

| Additiv (Produkt vom Beispiel) | Stabilisatormenge (Gew.-%) | Yellowness Index und Dauer der Hitzebelastung (h) | | | | |
|---|---|---|---|---|---|---|
| | | 0h | 1/2h | 1h | 1 1/2h | 2h |
| keines | – | 7 | 18 | 30 | 38 | 43 |
| 1 | 0,1 | 0 | 5 | 7 | 10 | 15 |
| 7 | 0,1 | 2 | 5 | 9 | 10 | 14 |

Beispiel 9

Stabilisierung von Polypropylen (Lichtstabilität)

Unstabilisiertes Polypropylenpulver (Hercules Profax® 6501) wird mit 0,2 Gew.-% eines der in der nachfolgenden Tabelle 2 angegebenen Additives gemischt. Die Mischung wird anschliessend bei 182 °C 5 Minuten lang auf einem Mischwalzwerk plastifiziert. Anschliessend wird die stabilisierte Polypropylenfolie vom Walzwerk abgelöst und abkühlen gelassen. Die Folie wird in Stücke geschnitten und auf einer hydraulischen Presse bei 220 °C und 12 bar zu einer 0,13 mm dicken Probe verformt. Diese Probe wird in einer Fluoreszensonnenlicht/Schwarzlichtkammer bis zur Zersetzung belichtet. Als Zersetzungszeitpunkt wird diejenige Anzahl Stunden gewertet, nach der die Probe die ersten Zerfallserscheinungen zeigt (braune Ecken, Risse). Die Messdaten sind in der nachfolgenden Tabelle 3 dargestellt.

Tabelle 3

| Additiv (Produkt vom Beispiel) | Zahl der Stunden bis zur Zersetzung |
|---|---|
| keines | 200–300 |
| 1 | 430 |
| 2 | 460 |
| 4 | 380 |
| 6 | 480 |
| 7 | 500 |

Beispiel 10

Stabilisierung von Polypropylen (Oxidationsstabilität)

Um die Oxidationsstabilität von Polypropylen, das 0,2 Gew.-% eines erfindungsgemässen Additivs oder einer synergistisch wirkenden Formulierung bestehend aus 0,1 Gew.-% eines Additivs und 0,3 Gew.-% Distearyldithiodipropionat (DSTDP) enthält, zu untersuchen, werden Plättchen von 0,64 mm Dicke hergestellt und in einem Umluftofen bei 150 °C gealtert. Als Zersetzungszeitpunkt wird diejenige Anzahl von Stunden gewertet, nach der die Probe die ersten Zerfallserscheinungen zeigt (braune Ecken, Risse).

Die Messdaten sind in Tabelle 4 dargestellt:

Tabelle 4

| Additiv (Produkt vom Beispiel) | Zahl der Stunden bis zur Zersetzung der Proben |
|---|---|
| keines | < 20 |
| 0,3% DSTDP | < 20 |

Tabelle 4 (Fortsetzung)

| Additiv (Produkt vom Beispiel) | Zahl der Stunden bis zur Zersetzung der Proben | |
|---|---|---|
| | 0,2% Additiv | 0,1% Additiv + 0,3% DSTDP |
| 1 | 20 | 80 |
| 2 | 30 | 160 |
| 4 | 50 | 220 |
| 6 | 20 | 280 |
| 7 | 120 | 180 |

**Patentansprüche**

1. Stoffzusammensetzung enthaltend ein gegen oxidativen, thermischen oder strahlungsinduzierten Abbau empfindliches organisches Material und eine stabilisierende Menge einer Verbindung der Formel I

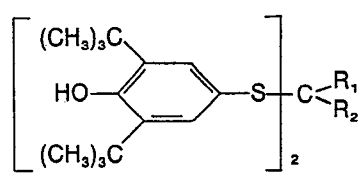

(I)

worin $R_1$ Wasserstoff oder $C_1$–$C_4$Alkyl ist, $R_2$ $C_6$–$C_8$Alkyl, Cyclohexyl, Cyclohex-3-enyl, Phenyl oder 3,5-Di-tert.-butyl-hydroxyphenyl bedeutet oder $R_1$ und $R_2$ zusammen $C_5$–$C_{11}$Alkylen bilden.

2. Stoffzusammensetzung gemäss Anspruch 1, dadurch gekennzeichnet, dass das organische Material ein synthetisches Polymere ist.

3. Stoffzusammensetzung gemäss Anspruch 2, dadurch gekennzeichnet, dass das synthetische Polymere ein homopolymeres oder copolymeres Polyolefin ist.

4. Stoffzusammensetzung gemäss Anspruch 1, dadurch gekennzeichnet, dass das organische Material Polystyrol, Acrylnitril/Butadien/-Styrol, Styrol/Butadien-Kautschuk, natürlicher Kautschuk oder ein Polyester ist.

5. Stoffzusammensetzung gemäss Anspruch 1, dadurch gekennzeichnet, dass in der Verbindung der Formel I $R_1$ Wasserstoff oder Methyl, $R_2$ $C_6$–$C_8$Alkyl oder $R_1$ und $R_2$ zusammen Pentamethylen sind.

6. Stoffzusammensetzung gemäss Anspruch 1, dadurch gekennzeichnet, dass die Verbindung der Formel I 2,2-Bis-(3,5-di-tert.-butyl-4-hydroxyphenylthio)octan ist.

7. Stoffzusammensetzung gemäss Anspruch 1, dadurch gekennzeichnet, dass die Verbindung der Formel I 1,1-Bis-(3,5-di-tert.-butyl-4-hydroxyphenylthio)-1-(3,5-di-tert.-butyl-4-hydroxyphenyl)-methan ist.

8. Stoffzusammensetzung gemäss Anspruch 1, dadurch gekennzeichnet, dass die Verbindung der Formel I 1,1-Bis-(3,5-di-tert.-butyl-4-hydroxyphenylthio)-cyclohexan ist.

9. Stoffzusammensetzung gemäss Anspruch 1, dadurch gekennzeichnet, dass die Verbindung der Formel I 1,1-Bis(3,5-di-tert.-butyl-4-hydroxyphenylthio)-1-(cyclohex-3-enyl)-methan ist.

10. Stoffzusammensetzung gemäss Anspruch 1, dadurch gekennzeichnet, dass die Verbindung der Formel I in einer Menge zwischen 0,01 und 5,0 Gew.%, bezogen auf das stabilisierte Material, enthalten ist.

11. Verbindung der Formel I

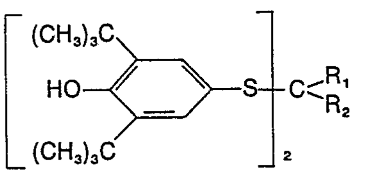

(I)

worin $R_1$ Wasserstoff und $R_2$ Cyclohexyl, Cyclohex-3-enyl oder 3,5-Di-tert.-butyl-4-hydroxyphenyl bedeuten.

## Claims

1. A composition of matter containing an organic material subject to oxidative, thermal or radiation-induced degradation and a stabilizing amount of a compound of formula I

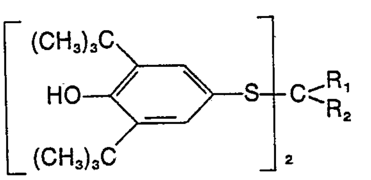

(I)

in which $R_1$ is hydrogen or $C_1$–$C_4$alkyl, $R_2$ is $C_6$–$C_8$alkyl, cyclohexyl, cyclohex-3-enyl, phenyl or 3,5-di-tert-butyl-4-hydroxyphenyl, or $R_1$ and $R_2$ together form $C_5$–$C_{11}$alkylene.

2. A composition of matter according to claim 1, wherein the organic material is a synthetic polymer.

3. A composition of matter according to claim 2, wherein the synthetic polymer is a homopolymeric or copolymeric polyolefin.

4. A composition of matter according to claim 1, wherein the organic material is polystyrene, acrylonitrile/butadiene/styrene, styrene/butadiene rubber, natural rubber or a polyester.

5. A composition of matter according to claim 1, wherein in the compound of fomrula I, $R_1$ is hydrogen or methyl, $R_2$ is $C_6$–$C_8$alkyl, or $R_1$ and $R_2$ together are pentamethylene.

6. A composition of matter according to claim 1, wherein the compound of formula I is 2,2-bis-(3,5-di-tert-butyl-4-hydroxyphenylthio)-octane.

7. A composition of matter according to claim 1, wherein the compound of formula I is 1,1-bis-(3,5-di-tert-butyl-4-hydroxyphenylthio)-1-(3,5-di-tert-butyl-4-hydroxyphenyl)-methane.

8. A composition of matter according to claim 1, wherein the compound of formula I is 1,1-bis-(3,5-di-tert-butyl-4-hydroxyphenylthio)-1-cyclohexane.

9. A composition of matter according to claim 1, wherein the compound of formula I is 1,1-bis-(3,5-di-tert-butyl-4-hydroxyphenylthio)-1-cyclohex-3-enyl)-methane.

10. A composition of matter according to claim 1 which contains the compound of formula I in an amount between 0.01 and 0.5% by weight, based on the stabilized material.

11. A compound of formula I

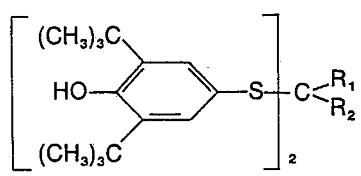

(I)

in which $R_1$ is hydrogen, and $R_2$ is cyclohexyl, cyclohex-3-enyl or 3,5-di-tert-butyl-4-hydroxyphenyl.

## Revendications

1. Composition de matières qui comprend une matière organique pouvant se dégrader par oxydation ou sous l'action de la chaleur ou d'un rayonnement, avec comme stabilisant un composé de formule I ci-dessous:

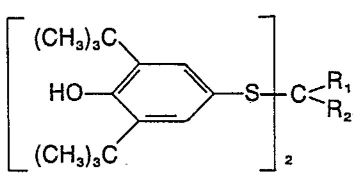

(I)

formule dans laquelle $R_1$ représente l'hydrogène ou un alkyle en $C_1$–$C_4$ et $R_2$ un alkyle en $C_6$–$C_8$ ou le groupe cyclohexyle, cyclohex-3-ényle, phényle ou 3,5-di-tert-butyl-4-hydroxy-phényle, ou encore $R_1$ et $R_2$ forment ensemble un alkylène en $C_5$–$C_{11}$.

2. Composition selon la revendication 1 caractérisée en ce que la matière organique est une matière polymère synthétique.

3. Composition selon la revendication 2 caractérisée en ce que le polymère synthétique est un homopolymère d'oléfine ou un copoolymère d'oléfines.

4. Composition selon la revendication 1 caractérisée en ce que la matière organique est du polystyrène, un copolymère d'acrylonitrile, de butadiène et de styrène, un caoutchouc de styrène et de butadiène, du caoutchouc naturel ou un polyester.

5. Composition selon la revendication 1 caractérisée en ce que, dans le composé de formule I, $R_1$ est l'hydrogène ou le groupe méthyle et $R_2$ un alkyle en $C_6$–$C_8$, ou bien $R_1$ et $R_2$ forment ensemble un groupe pentaméthylène.

6. Composition selon la revendication 1 caractérisée en ce que le composé de formule I est le 2,2-bis-(3,5-di-tert-butyl-4-hydroxyphénylthio)octane.

7. Composition selon la revendication 1, caractérisée en ce que le composé de formule I est le 1,1-bis-(3,5-di-tert-butyl-4-hydroxyphénylthio)-1-(3,5-di-tert-butyl-4-hydroxyphényl)-méthane.

8. Composition selon la revendication 1 caractérisée en ce que le composé de formule I est le 1,1-bis-(3,5-di-tert-butyl-4-hydroxyphénylthio)-cyclohexane.

9. Composition selon la revendication 1 caractérisée en ce que le composé de formule I est le 1,1-bis-(3,5-di-tert-butyl-4-hydroxyphénylthio)-1-(cyclohex-3-ényl)-méthane.

10. Composition selon la revendication 1 caractérisée en ce que la proportion du composé de formule I est de 0,01 à 5,0% du poids de la matière stabilisée.

11. Les composés de formule I ci-dessous:

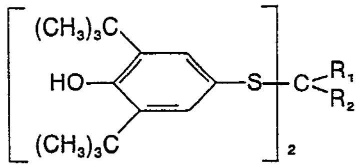

(I)

dans laquelle $R_1$ représente l'hydrogène et $R_2$ le groupe cyclohexyle, cyclohex-3-ényle ou 3,5-di-tert-butyl-4-hydroxyphényle.